# EUROPEAN PATENT APPLICATION

(11) **EP 4 613 294 A1**
(43) Date of publication of application: **10.09.2025**
(21) Application number: 24161804.0
(22) Date of filing: 06.03.2024
(51) Int. Cl.: A61L 2/04, A61L 11/00, B65B 69/00, B26D 1/00, B26D 1/143, B26D 7/01, B26D 7/02

(54) **DEVICE FOR AUTOMATIC EMPTYING OF URINE BAGS AND APPARATUS INCLUDING IT**

(71) Applicant: Bonferraro S.p.A., 37060 Bonferraro (VR) (IT)
(72) Inventor: OLIVIERI, Martino, Verona VR (IT); LOCATELLI, Andrea, Verona VR (IT); GOBBI, Ezio, Roncoferraro MN (IT)
(74) Representative: Concone, Emanuele

(57) **Abstract**

A device for clamping and cutting a urine bag includes a frame (3) configured to be attached to the door (2) of a washing and thermodisinfection apparatus, a holder (4) mounted on the frame (3), a retainer (5) rotating between a bag loading/unloading position and a bag clamping position, blades (12, 13) movable between a rest position and a bag cutting position, and at least one actuator (6) carrying the blades (12, 13) and connected to the retainer (5) via a rod (9) pivoted between the retainer (5) and the actuator (6), which is mounted on the frame (3) via a rotation fulcrum (7) so that when the door (2) is open the weight of the actuator (6) keeps the retainer (5) in the loading/unloading position and the blades (12, 13) in the rest position, and when the door (2) is closed the retainer (5) is in the clamping position and the blades (12, 13) are in the cutting position.

## Description

The present invention relates to apparatuses for the emptying and thermodisinfection of bags used in medical settings to collect urine from bedridden patients, and in particular to a device for receiving a bag into the apparatus and cutting it automatically to cause it to be emptied.

It is known that urine bags must be emptied and thermodisinfected before they are discarded, and there are washing and thermodisinfection apparatuses for this purpose equipped with a device mounted on the door that receives the bag. When the door is closed and the bag is inside the wash tank, the device cuts it off at the bottom to allow it to be emptied by gravity and properly washed, so that there is no risk of contamination to the operator when he or she removes the emptied bag from the apparatus.

To prevent the bag from moving from its loading position during door closure, with the risk of falling into the wash tank or otherwise ending up in a position where cutting is not possible or not entirely effective, the device includes a bag clamping element, which typically also performs a crushing of the top of the bag to push all the contents to the bottom where cutting is performed. This clamping element is manually operated by the operator, who must either overcome the resistance of a spring biasing it to the clamping position, or must engage and disengage the element when loading and unloading the bag (with the risk of not engaging the element properly and allowing the bag to shift).

Regarding the blades that cut the bag, in known devices they are of two types: progressively actuated or snap-actuated. Progressively actuated blades are typically a pair of blades arranged in a scissor pattern between which the bottom of the bag is placed, so that when the door is closed, the blades are automatically actuated to cut and empty the bag. This solution implies that the blades are accessible to the operator when loading the bag, with the risk that a finger could be cut in placing the bag. In addition, a progressive scissor cut does not guarantee high effectiveness because the bag could slip between the blades and get stuck without being cut or be cut only partially, resulting in difficulty in emptying.

The snap blades are automatically actuated by one or more springs when the door is closed, so that they are triggered from a rest position to an operational position where they cut the bag. This solution provides greater assurance of cutting effectiveness, but also implies less reliability because repeated heating cycles for thermodisinfection affect the strength of the springs over time. Moreover, when unloading the bag, the operator not only has to open the clamping element but also has to reload the springs by manually returning the blades to their rest position.

It is therefore an object of the present invention to provide a device that overcomes these drawbacks. Said object is achieved by means of a device that uses the opening and closing motion of the apparatus door to achieve movement of the blades and of the clamping element by a combined action of gravity and of a contrasting surface on a drive element. Other advantageous features of the present device are specified in the dependent claims.

Thus, the main advantage of the device according to the present invention is that it does not require any manual action by the operator beyond placing and removing the bag.

A second significant advantage is to be as simple and reliable as a progressive device while providing the effectiveness and operational safety of a snap-actuated device, with no risk to the operator of coming into contact with the blades.

Another important advantage of this device comes from its simple structure, with reduced number of components, which allows it to be made at a low cost. It is therefore possible to offer it as an upgrade, so that it can be included in apparatuses that had been equipped with a prior art device at the time of installation, with an advantage also from a marketing point of view.

These and other advantages and features of the device according to the present invention will be evident to those skilled in the art from the following detailed description of an embodiment thereof, with reference to the attached drawings in which:
Fig.1 is a top view of the device mounted on the open door of an apparatus and with a bag loaded on the device;
Fig.2 is a cross-sectional side view along line II-II of Fig.1, but without the bag and with an enlarged detail;
Fig.3 is an enlarged view of the device in Fig.2;
Fig.4 is a side view in transparency of the device in Fig. 1 with the door in an intermediate position;
Fig.5 is a sectional view along line V-V of Fig.4;
Fig.6 is a side view in transparency of the apparatus of Fig.1 with the door in a closed position;
Fig.7 is a sectional view along line VII-VII of Fig.6; and
Fig.8 is a perspective view of the device in the rest position as in Fig.2.

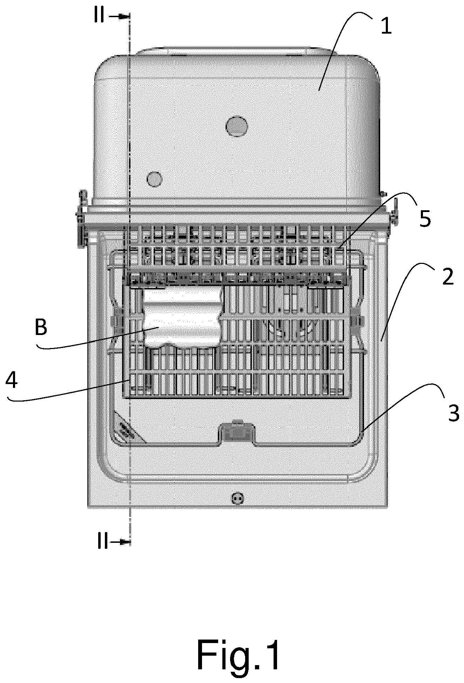

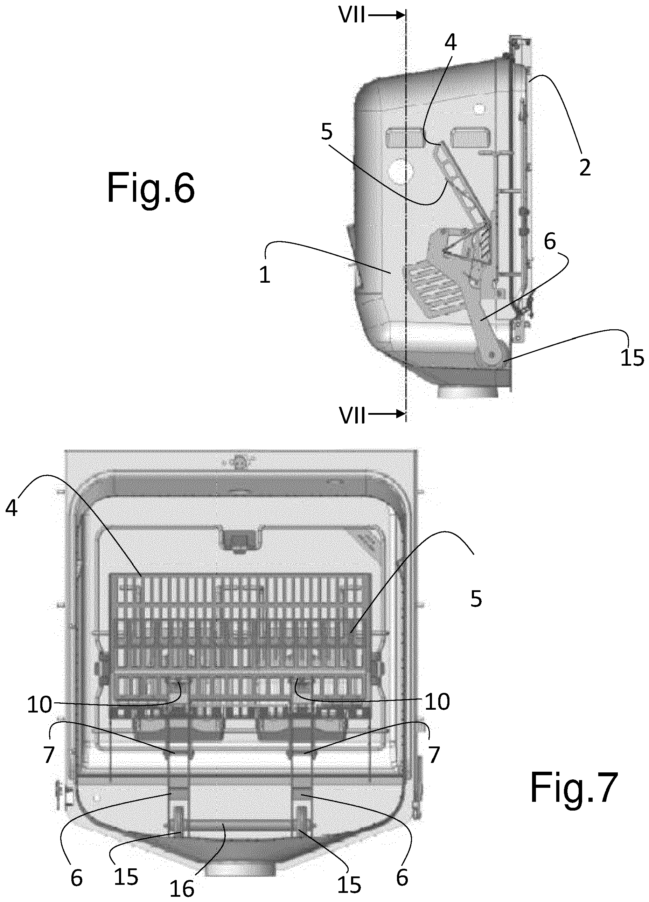

Referring to the above figures, it can be seen that a urine bag emptying and thermodisinfection apparatus traditionally includes a wash tank 1 closed at the front by a door 2 hinged at the bottom so that it rotates between an open position in which it is arranged almost horizontally (Figs.1-2) and a closed position in which it is arranged vertically (Figs.6-7). A device is mounted on door 2 that receives a bag B so that it can be easily loaded when the door is open, and then clamped and cut so that when the door is closed, bag B is emptied and arranged in the wash tank 1 (Figs.6-7). For this purpose, the device includes a frame 3 configured to be attached to door 2 and a bag holder 4 mounted on frame 3, as well as a clamping element or retainer 5 typically mounted on holder 4 so as to rotate between a bag loading/unloading position and a bag clamping position.

As mentioned above, a first novel aspect of the device according to the present invention resides in the automatic rotation of retainer 5 during the closing and opening motion of door 2, exploiting only gravity and at least one drive element or actuator 6 (two in the illustrated example) rotatably mounted on frame 3 via a rotation fulcrum 7. Holder 4 comprises an inclined slide-like outer part 4a on which the operator places bag B, and a substantially L-shaped inner part 4b that serves as a support for bag B. Retainer 5 is mounted to the top of part 4b of support 4 via a pair of hinges 8, and is moved by actuator 6 via a rod 9 pivoted between said elements 5 and 6.

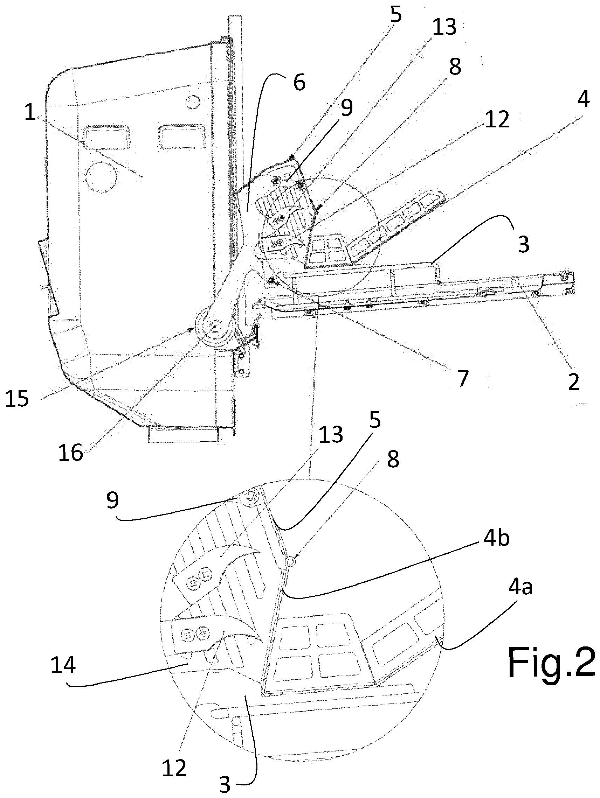

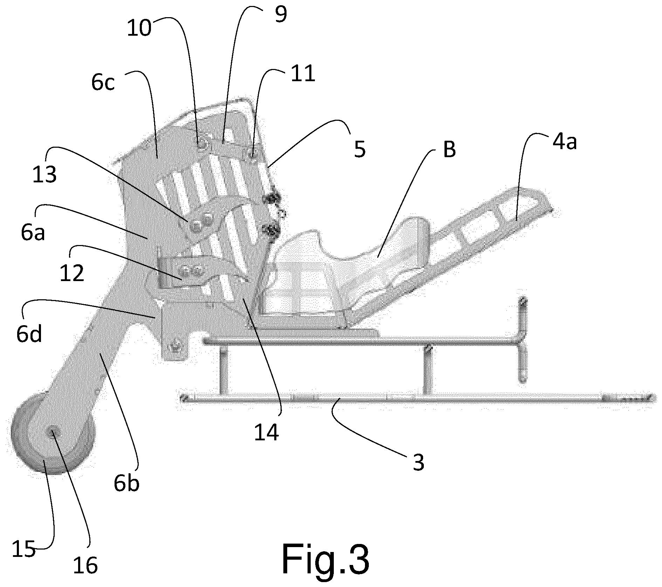

More specifically, as shown in detail in figures 2 and 3, actuator 6 comprises an upper portion 6a and a lower portion 6b, with a first upwardly inclined frontal extension 6c formed at the top of portion 6a, and a second downwardly inclined frontal extension 6d formed at the top of portion 6b. A first pin 10 connects the end of extension 6c to rod 9, which is connected to retainer 5 by a second pin 11. Rotation fulcrum 7 is placed at the inner end of frame 3, and actuator 6 is connected thereto at the end of extension 6d.

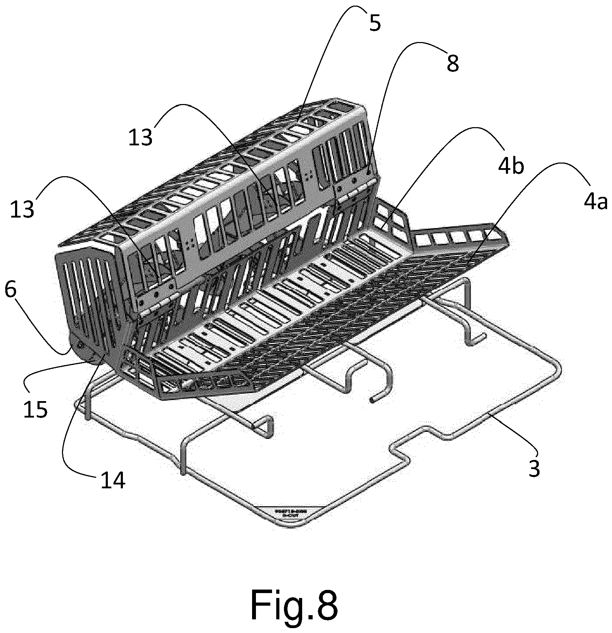

A plurality of lower blades 12 and a plurality of upper blades 13 are mounted frontally on the upper portion 6a, only one blade of each set of blades being visible in the figures, and in the loading/unloading position they are inaccessible since they are protected superiorly by retainer 5, frontally by portion 4b of holder 4, and preferably also laterally by sidewalls 14 (see also Fig.8). All these protective elements are provided with openings for the passage of washing liquid, and in portion 4b these openings are configured to also allow the passage of blades 12, 13 as described below.

A wheel 15 is preferably mounted at the base of portion 6b via a pivot 16 that can also incorporate a counterweight. The purpose of wheel 15 is to allow actuator 6 to slide with minimal friction against the front wall of the wash tank 1, and the purpose of counterweight 16 is to gravity hold actuator 6 in the loading/unloading position with retainer 5 folded over blades 12, 13 when door 2 is open. Note that these functions may also be performed by equivalent elements, or these elements may even be absent, e.g., wheel 15 may be replaced by a skid made of a low-friction material, or counterweight 16 may not be needed if the weight of wheel 15 and/or portion 6b is sufficient to keep actuator 6 in the desired position.

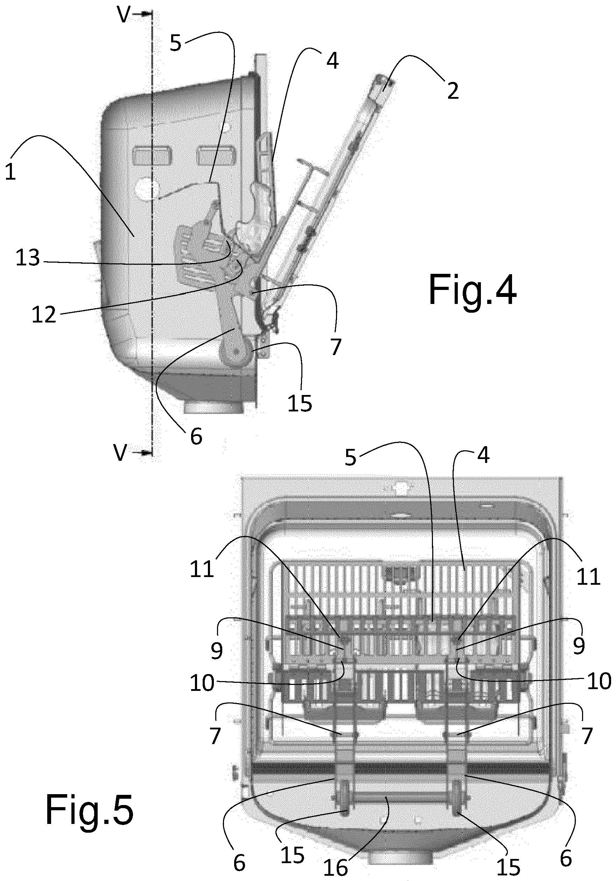

In the exemplary embodiment shown in the figures, with particular reference to figures 5 and 7, it can be seen that two "double" actuators 6 are provided, each equipped with a wheel 15 centrally arranged between the two flanks of actuator 6, and with a counterweight 16 extending between the two actuators 6 and acting as a pivot for both wheels 15. Similarly, there are two rotation fulcrums 7 each extending between the two flanks of each actuator 6, and the same is true for pivots 10 connecting actuators 6 to rods 9.

Note that this is the preferred solution for balanced operation of the device and to be able to effectively cut bag B regardless of the position in which it is placed by the operator, even being able to load two bags side by side if they are as small as the one depicted in the drawings. However, it would also be possible to use a single central actuator or a larger number of actuators, always preferably arranged symmetrically with respect to the midplane of the device.

In the light of the above description, the simple and effective operation of the device according to the invention is immediately understandable.

In the open position of door 2 illustrated in figures 1-3, the operator can easily load bag B by simply placing it on holder 4, since retainer 5 is held open by gravity by actuator 6. While lifting door 2 to close the apparatus, when door 2 reaches an inclination such that wheel 15 at the end of the lower portion 6b makes contact with the front wall of the wash tank 1, which acts as a contrasting surface, actuator 6 cannot rotate further together with frame 3 therefore it begins to rotate relative thereto around fulcrum 7.

As a result, retainer 5 is pushed by rod 9 to rotate forward relative to holder 4, and blades 12, 13 begin to protrude through part 4b so that bag B is "clawed" and retained even before it is clamped by retainer 5 (Figs.4-5). When door 2 reaches the closed position, retainer 5 has completed its rotation around hinge 8 abutting against part 4a and crushing the upper portion of the bag, while blades12, 13 have torn the bag in several places ensuring its emptying (Figs.6-7). Note that in the transition from the intermediate to the closed position of door 2, actuator 6 does not rotate further relative to the apparatus but slides downward to the bottom of the wash tank.

At the end of the washing and thermodisinfection cycle, the operator opens door 2, and the movement of the device is practically the reverse of that described above, with actuator 6 sliding up along the front wall of the wash tank 1 until fulcrum 7 pulls it into rotation, while rod 9 returns retainer 5 to the unloading position allowing easy removal of the emptied and thermodisinfected bag without risk of contact with the blades.

It is clear that the embodiment of the device according to the invention described and illustrated above is only an example susceptible to many variations. In particular, the exact type and arrangement of the elements could be varied according to specific constructional requirements, realizing variations that are within the reach of a person skilled in the art based on the description given above.

## Claims

1. A device for clamping and cutting a urine bag (B), comprising a frame (3) configured to be attached to the door (2) of a washing and thermodisinfection apparatus, a holder (4) mounted on said frame (3) and configured to receive said bag (B), a retainer (5) configured to rotate between a position for loading/unloading the bag (B) and a position for clamping the bag (B), and a plurality of blades (12, 13) configured to move between a rest position and a bag (B) cutting position, **characterized in that** it includes at least one actuator (6) that carries said blades (12, 13) and is connected to said retainer (5) via a rod (9) pivoted between said retainer (5) and said actuator (6), which is mounted on said frame (3) by means of a rotation fulcrum (7) arranged on the frame (3) in such a position that when the device is in a substantially horizontal position the weight of the actuator (6) keeps the retainer (5) in said bag (B) loading/unloading position and the blades (12, 13) in said rest position, and when the device is in a substantially vertical position the retainer (5) is in said clamping position and the blades (12, 13) are in said cutting position, **and in that** the blades (12, 13) are protected by the holder (4) and the retainer (5) in the rest position and pass through appropriate openings in the holder (4) to reach the cutting position.

2. Device according to claim 1, **characterized in that** the actuator (6) is equipped at its lower end with a wheel (15) and/or a counterweight (16).

3. Device according to claim 1 or 2, **characterized in that** it comprises two or more actuators (6) arranged symmetrically with respect to the midplane of the device.

4. Device according to any of the preceding claims, **characterized in that** it comprises sidewalls (14) protecting the blades (12, 13) in the rest position.

5. Device according to any of the preceding claims, **characterized in that** the actuator (6) comprises an upper portion (6a) and a lower portion (6b), with a first frontal extension (6c) sloping upwardly formed at the top of said upper portion (6a), and a second frontal extension (6d) sloping downwardly formed at the top of said lower portion (6b), a pin (10) for connection to the rod (9) being arranged at the end of said first frontal extension (6c), and the rotation fulcrum (7) being connected to the actuator (6) at the end of said second frontal extension (6d).

6. A washing and thermodisinfection apparatus comprising a wash tank (1) closed frontally by a door (2) hinged inferiorly, so as to rotate between an open position in which it is disposed nearly horizontally and a closed position in which it is disposed vertically, **characterized in that** it includes a device according to any of the preceding claims attached to the door (2).
